(19) Europäisches Patentamt
European Patent Office
Office européen des brevets

(11) EP 4 789 662 A1

(12) **EUROPEAN PATENT APPLICATION**
published in accordance with Art. 153(4) EPC

(43) Date of publication:
**12.08.2026 Bulletin 2026/33**

(21) Application number: **24874807.1**

(22) Date of filing: **22.10.2024**

(51) International Patent Classification (IPC):
***A61K 9/72*** (2006.01) ***A61K 9/14*** (2006.01)
***A61K 9/16*** (2006.01) ***A61K 31/41*** (2006.01)
***A61K 31/522*** (2006.01) ***A61K 38/26*** (2006.01)
***A61K 39/395*** (2006.01) ***A61K 45/00*** (2006.01)
***A61K 47/26*** (2006.01) ***A61P 3/10*** (2006.01)
***A61P 11/00*** (2006.01) ***A61P 11/02*** (2006.01)
***A61P 11/06*** (2006.01) ***A61P 35/00*** (2006.01)
***A61P 37/02*** (2006.01) ***A61P 37/08*** (2006.01)
***A61P 43/00*** (2006.01)

(52) Cooperative Patent Classification (CPC):
**A61K 9/0073; A61K 9/14; A61K 9/16; A61K 31/41; A61K 31/522; A61K 38/26; A61K 39/395; A61K 45/00; A61K 47/26; A61P 3/10; A61P 11/00; A61P 11/02; A61P 11/06; A61P 35/00; A61P 37/02;**
(Cont.)

(86) International application number:
**PCT/JP2024/037613**

(87) International publication number:
**WO 2025/075208 (10.04.2025 Gazette 2025/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.10.2023 JP 2023173648**
**27.06.2024 JP 2024103674**

(71) Applicants:
- **Nagase Viita Co., Ltd.**
**Okayama-shi, Okayama 702-8006 (JP)**
- **Shizuoka Prefectural University Corporation**
**Shizuoka-shi, Shizuoka 422-8526 (JP)**

(72) Inventors:
- **ONOUE, Satomi**
**Shizuoka-shi, Shizuoka 422-8526 (JP)**
- **HASHIMOTO, Kotaro**
**Okayama-shi, Okayama 702-8006 (JP)**
- **ARIYASU, Toshio**
**Okayama-shi, Okayama 702-8006 (JP)**
- **KONO, Keizo**
**Okayama-shi, Okayama 702-8006 (JP)**

(74) Representative: **Vossius & Partner Patentanwälte Rechtsanwälte mbB**
**Siebertstraße 3**
**81675 München (DE)**

(54) **POWDER FOR INHALATION**

(57) Provided is an inhalation powder capable of maintaining the pharmacological activity of an active pharmaceutical ingredient while exhibiting excellent lung delivery efficiency. The inhalation powder includes carrier particles (a) and fine particles (b) present on surfaces of the carrier particles, the fine particles (b) including an active pharmaceutical ingredient and an excipient, the excipient including trehalose.



Processed by Luminess, 75001 PARIS (FR)

(Cont. next page)

FIG.3

mT/PL-RP (Example 1)
49.7%
PL deposited
(% of total amount)
0
5
10
15
20
25
30
#0
#1
#2
#3
#4
#5
#6
#7
Stages in cascade impactor

L/PL-RP (Comparative Example 1)

37.5%
PL deposited
(% of total amount)
0
5
10
15
20
25
30
#0
#1
#2
#3
#4
#5
#6
#7
Stages in cascade impactor

(52) Cooperative Patent Classification (CPC): (Cont.)
**A61P 37/08; A61P 43/00**

## Description

TECHNICAL FIELD

The present invention relates to inhalation powders.

BACKGROUND ART

Pulmonary drug administration is considered promising as it can bypass the hepatic first-pass effect, leading to lower dosing and a corresponding decrease in adverse effects. Examples of preparations for pulmonary administration include inhalation powders (dry powder inhaler (DPI)) and inhalation liquids. Of these, inhalation powders are excellent in that they have a higher storage stability than inhalation liquids and can be inhaled without an equipment such as a nebulizer.
A known inhalation powder includes carrier particles having a diameter of about 50 μm to 125 μm and fine particles having a diameter of about 1 μm to 5 μm present on the surfaces of the carrier particles, the fine particles consisting of a mixture of an active pharmaceutical ingredient and an auxiliary substance (excipient). It is known that lactose can be used as the excipient (Patent Literature 1).

CITATION LIST

- Patent Literature

Patent Literature 1: JP 2001-072586 A

SUMMARY OF INVENTION

- Technical Problem

Lactose, which has been used as an excipient, is a reducing sugar. Thus, there is a concern that lactose may react with an active pharmaceutical ingredient such as a peptide or a protein contained in a preparation, posing a risk of reducing the stability of the preparation. The present invention aims to provide an inhalation powder capable of maintaining the pharmacological activity of an active pharmaceutical ingredient while exhibiting excellent lung delivery efficiency.

- Solution to Problem

The present inventors have found that the above problem can be solved by a specific inhalation powder. The inhalation powder includes carrier particles and fine particles present on the surfaces of the carrier particles, the fine particles including an active pharmaceutical ingredient and an excipient which is not a reducing sugar but is trehalose having excellent crystallinity. Thus, the present invention has been completed.
The present disclosure may include the following embodiments.
(Clause 1) An inhalation powder, including:

carrier particles (a); and
fine particles (b) present on surfaces of the carrier particles, the fine particles (b) including an active pharmaceutical ingredient and an excipient,
the excipient including trehalose.

(Clause 2) The inhalation powder according to clause 1,
wherein the fine particles (b) have a mean particle size of 0.8 μm or more and 5.0 μm or less.
(Clause 3) The inhalation powder according to clause 1 or 2,
wherein the carrier particles (a) include at least one of lactose or trehalose.
(Clause 4) The inhalation powder according to clause 1 or 2,
wherein the carrier particles (a) have a mean particle size of 30 μm or more and 300 μm or less.
(Clause 5) The inhalation powder according to clause 1 or 2,
wherein the active pharmaceutical ingredient is selected from the group consisting of a low molecular weight compound, a peptide, and a protein.
(Clause 6) The inhalation powder according to clause 5,
wherein the low molecular weight compound is selected from the group consisting of a leukotriene receptor antagonist and

a xanthine derivative.

(Clause 7) The inhalation powder according to clause 6,
wherein the leukotriene receptor antagonist is pranlukast, or the xanthine derivative is theophylline.

(Clause 8) The inhalation powder according to clause 5,
wherein the peptide or the protein is glucagon or an antibody.

(Clause 9) The inhalation powder according to clause 1 or 2,
wherein the inhalation powder has a total deposition rate in stages #4 to #7 of 10% or more in an inhalation performance evaluation using an Andersen cascade impactor.

(Clause 10) The inhalation powder according to clause 1 or 2,
wherein the fine particles (b) are prepared by drying a mixture of the active pharmaceutical ingredient and the excipient.

(Clause 11) The inhalation powder according to clause 1 or 2,
wherein the weight ratio of the active pharmaceutical ingredient and the excipient is 1:1999 to 19:1.

(Clause 12) The inhalation powder according to clause 1 or 2,
wherein the weight ratio of the carrier particles (a) and the fine particles (b) is 1:1 to 99:1.

- Advantageous Effects of Invention

The inhalation powder of the present invention features low reactivity between the excipient and the active pharmaceutical ingredient and is excellent in lung delivery efficiency.

## BRIEF DESCRIPTION OF DRAWINGS

FIG. 1 shows a scanning electron microscopic image of micronized trehalose.

FIG. 2 shows scanning electron microscopic images of inhalation powders.

FIG. 3 shows the inhalation performances of inhalation powders in Example 1 and Comparative Example 1 evaluated using an Andersen cascade impactor.

FIG. 4 shows the inhalation performances of inhalation powders in Examples 1 and 2 evaluated using an Andersen cascade impactor.

FIG. 5 shows particle size distributions of fine particles (b) including an active pharmaceutical ingredient and an excipient in Examples 1 and 2 and Comparative Example 1.

FIG. 6 shows the inhalation performance of the inhalation powder in Example 1 evaluated using an Andersen cascade impactor after a storage stability test.

FIG. 7 shows lung tissues after administering a preparation in Example 1 or Comparative Example 2.

FIG. 8 shows the number of infiltrated cells in a bronchoalveolar lavage fluid after administering the preparation in Example 1 or Comparative Example 2.

FIG. 9 shows the amounts of the active pharmaceutical ingredient transferred into blood after administering the preparation in Example 1 or Comparative Example 2.

FIG. 10 shows the inhalation performances of inhalation powders in Example 3 and Comparative Example 3 evaluated using an Andersen cascade impactor.

FIG. 11 shows the inhalation performances of inhalation powders in Example 4 and Comparative Example 4 evaluated using an Andersen cascade impactor.

## DESCRIPTION OF EMBODIMENTS

The inhalation powder of the present invention includes carrier particles (a) and fine particles (b) present on surfaces of the carrier particles, the fine particles (b) including an active pharmaceutical ingredient and an excipient, the excipient including trehalose.

<Carrier particles (a)>

The carrier particles (a) can improve the inhalation efficiency of the inhalation powder. Moreover, when the fine particles (b) including an active pharmaceutical ingredient and an excipient adhere to the surfaces of the carrier particles (a), the carrier particles (a) can prevent the active pharmaceutical ingredient from aggregating, thereby allowing for stable dispersion. To enhance the absorption efficiency of the active pharmaceutical ingredient in the lungs or bronchi, preferably, the carrier particles (a) efficiently separate from the fine particles (b) including an active pharmaceutical ingredient and an excipient upon inhalation of the inhalation powder. After the carrier particles (a) are separated from the fine particles (b)

including an active pharmaceutical ingredient and an excipient, the carrier particles (a) preferably deposit in the upper respiratory tract and are subsequently cleared from the body.

The carrier particles (a) have a mean particle size of preferably 30 μm or more and 300 μm or less, more preferably 30 μm or more and 100 μm or less, still more preferably 40 μm or more and 80 μm or less. When the mean particle size is 30 μm or more, the deposition of the carrier particles (a) in the upper respiratory tract and clearance from the body tend to be promoted. When the mean particle size is 300 μm or less, the inhalation powder tends to be easily inhaled. Herein, the mean particle size refers to a particle size (Dv50) corresponding to 50% of the cumulative volume-based particle size distribution. The mean particle size can be measured with a light scattering particle size analyzer (for example, Mastersizer 3000 available from Malvern Panalytical).

The carrier particles (a) may be made of any material that has been used as a carrier in inhalation powders. Examples of usable materials include saccharides, sugar alcohols, and other common excipients. Examples of the saccharides include lactose, trehalose, glucose, fructose, sucrose, maltose, cellobiose, and maltotriose. Examples of the sugar alcohols include erythritol, sorbitol, mannitol, maltitol, xylitol, arabitol, dulcitol, palatinose, lactitol, inositol, and xylose. Examples of the common excipients include calcium sulfate, calcium carbonate, calcium phosphate, talc, titanium oxide, crystalline cellulose, starches, and dextran. Of these, saccharides are preferred, and lactose and trehalose are more preferred. Each of the above materials may be used alone, or two or more of these may be used in combination. For example, the carrier particles (a) may include a mixture of lactose and trehalose.

The percentage of the carrier particles (a) in the inhalation powder is preferably 50 to 99% by weight, more preferably 80 to 95% by weight, still more preferably 85 to 90% by weight. When the percentage is within the above ranges, the fine particles (b) tend to uniformly adhere to the surfaces of the carrier particles (a) while maintaining the pharmacological activity.

<Active pharmaceutical ingredient>

The active pharmaceutical ingredient contained in the fine particles (b) may be any active pharmaceutical ingredient (API) usable in inhalation powders. Examples include a low molecular weight compound, a peptide, a protein, a nucleic acid, a sugar, and a lipid (lipid nanoparticles). Examples of the low molecular weight compound include a leukotriene receptor antagonist such as pranlukast or montelukast; a xanthine derivative such as caffeine, theophylline, or theobromine; a β2 agonist such as salmeterol, formoterol, or indacaterol; an anticholinergic drug such as tiotropium, acridinium, or umeclidinium; and a steroid such as fluticasone, budesonide, or mometasone.

Any peptide or protein formed by bonding multiple amino acids via peptide bonds may be used. Examples include an antibody, a hormone, a cytokine, a chemokine, an enzyme, and an enzyme inhibitor. Examples of the peptide or the protein are described below. They may be those with modifications such as formation of intramolecular disulfide bonds or cyclization or those with addition of groups such as polyethylene glycol groups, fatty acid groups, or phosphate groups. They may also be viral structural proteins or inactivated or attenuated viruses.

The antibody may be any antibody capable of specifically binding to an antigen and may be any of IgG, IgA, IgM, IgD, and IgE. Examples of the IgG include IgG1, IgG2, IgG3, and IgG4. Examples of the IgA include IgA1 and IgA2. The origin of the antibody may be any animal, and examples of the animal include humans and animals other than humans such as mice, rats, rabbits, sheep, goats, and chickens. The antibody may be a chimeric antibody or a humanized antibody, each consisting of sequences derived from a non-human animal and sequences derived from a human, or may be a fully human antibody. Examples of the antibody include not only an antibody including an intact constant region and an intact variable region, but also Minibody, Fab, Diabody, Fab', Triabody, scFv-Fc, scFv, $F(ab')_2$, and Fv. The antibody may also be a conjugated antibody obtained by conjugating a compound other than antibodies, such as a nucleic acid, a labeled compound, or a low molecular weight drug, to an antibody. Non-limiting examples of the antigen to which the antibody specifically binds include a nucleic acid, a protein, and a sugar. Specific examples of the antigen include IFNγ, TNFα, IL-1, IL-4, IL-5, IL-6, IL-7, IL-13, IL-18, and a leukocyte surface antigen protein.

Examples of the hormone include glucagon, insulin, a parathyroid hormone (PTH), a gonadotropin, a growth hormone, an adrenal medullary hormone, an adrenocortical hormone, calcitonin, melatonin, prolactin, oxytocin, inhibin, an amine/amino acid-derived hormone, a steroid hormone, and somatostatin.

Examples of the cytokine include IFN such as IFNα, IFNβ, IFNγ, IFNδ, IFNω, IFNλ, IFNε, or IFNκ; TNF such as TNFα or TNFβ; interleukin (IL) such as IL-1, IL-2, IL-3, IL-4, IL-5, IL-6, IL-7, IL-8, IL-9, IL-10, IL-11, IL-12, IL-13, IL-14, IL-15, IL-16, IL-17, or IL-18; CSF, EPO, EGF, FGF, and PDGF.

Examples of the chemokine include CC chemokine such as TCA3, MCP1, MCP2, MCP3, MCP4, MCP5, MIP4, MDC, or MEC, and CXC chemokine such as IL-8, MIG, IP10, PBSF, BLC, NAP3, MIP2, or PF4.

Any nucleic acid formed by bonding multiple nucleotides and/or nucleosides via phosphodiester bonds may be used, and examples of the nucleic acid include DNA, RNA, mRNA, and siRNA. The nucleic acid may include an artificially modified nucleotide and/or nucleoside, such as LNA.

Of these active pharmaceutical ingredients, a low molecular weight compound, a peptide, or a protein is

preferred; a leukotriene receptor antagonist, a xanthine derivative, a peptide, or a protein is more preferred; and pranlukast, theophylline, glucagon, or an antibody is still more preferred.

The molecular weight of the active pharmaceutical ingredient is not limited. In the case of a low molecular weight compound, the molecular weight is preferably 100 or more and 1000 or less, more preferably 150 or more and 600 or less. In the case of an active pharmaceutical ingredient other than low molecular weight compounds, the molecular weight is preferably 1000 or more and 1000000 or less, more preferably 2000 or more and 200000 or less. The active pharmaceutical ingredient in the inhalation powder of the present invention can be efficiently delivered to the bronchi or the lungs, even if it has a large molecular weight.

<Excipient>

Trehalose is used as the excipient in the fine particles (b). The trehalose may be a derivative of trehalose in which hydrogen atoms of some hydroxy groups are replaced by substituents. Examples of the substituents include a carboxylic acid ester such as acetate or benzoate; a sulfuric acid ester; a fatty acid ester such as laurate, myristate, palmitate, stearate, oleate, linoleate, or linolenate; and an ether such as methyl ether, benzyl ether, trityl ether, methylsilyl ether, or dodecyl ether.

A common commercially available trehalose (mean particle size: about 300 μm) as it is may be used as the excipient in the inhalation powder. The mean particle size of non-micronized trehalose is preferably 30 μm or more and 300 μm or less. The trehalose may be micronized trehalose, and the mean particle size of micronized trehalose is preferably 0.5 μm or more and 8.0 μm or less, more preferably 0.8 μm or more and 3.0 μm or less. Here, non-limiting examples of the micronization method include chemical treatment (e.g., dissolution, reprecipitation) and physical treatment (e.g., ultrasonication, grinding, granulation).

In addition to trehalose, the excipient may include a second excipient consisting of a different substance other than trehalose. Examples of the second excipient include a saccharide such as lactose, glucose, or sucrose; a sugar alcohol such as erythritol, mannitol, or sorbitol; a starch; a high molecular weight polymer such as cellulose, methylcellulose, hydroxypropylcellulose, hydroxypropylmethylcellulose, carmellose sodium, pullulan, dextrin, gum arabic, agar, gelatin, tragacanth, sodium alginate, polyvinylpyrrolidone, or poly(vinyl alcohol); a fatty acid or a salt thereof; a wax; calcium sulfate; calcium carbonate; talc; titanium oxide; and light anhydrous silicic acid. In the case where the excipient includes the second excipient other than trehalose, the amount of the trehalose in the excipient is preferably 1.0% by weight or more and 99.9% by weight or less, more preferably 50% by weight or more and 95% by weight or less.

<Fine particles (b)>

Preferably, the active pharmaceutical ingredient and the excipient in the fine particles (b) are both in the form of fine particles and uniformly mixed with each other. Such fine particles (b) can be obtained by blending and/or grinding the active pharmaceutical ingredient and the excipient. The active pharmaceutical ingredient and the excipient can be blended with each other or ground using a common dry grinder. Examples of the dry grinder include a jet mill, a media agitation mill, a high-speed rotary friction mill, a high-speed rotary impact mill, an impact mill, a rolling ball mill, a centrifugal ball mill, a vibration ball mill, and a planetary ball mill. A jet mill is preferred among these. When using a jet mill, the feeding pressure is preferably 4.0 $kg/cm^2G$ or more and 9.0 $kg/cm^2G$ or less, more preferably 6.0 $kg/cm^2G$ or more and 7.0 $kg/cm^2G$ or less. The grinding pressure is preferably 3.5 $kg/cm^2G$ or more and 8.5 $kg/cm^2G$ or less, more preferably 5.5 $kg/cm^2G$ or more and 6.5 $kg/cm^2G$ or less. Examples of the method for blending the active pharmaceutical ingredient and the excipient include freeze-drying or spray-drying a liquid in which they are blended, thereby preparing the fine particles (b).

The active pharmaceutical ingredient and the excipient may be freeze-dried before they are treated with a dry grinder. The freeze-drying enables preparation of a dry powder in which the active pharmaceutical ingredient and the excipient are uniformly mixed at a molecular level. The freeze-drying is preferably performed by uniformly mixing the active pharmaceutical ingredient and the excipient in a solvent, then freezing the mixture preferably at -80°C or higher and 0°C or lower, more preferably at -80°C or higher and -20°C or lower, and drying the frozen mixture in vacuum. Examples of usable solvent include water, a buffer, physiological saline, Ringer's solution, an organic solvent, and a liquid mixture of these.

The weight ratio of the active pharmaceutical ingredient and the excipient in the fine particles (b) is preferably 1:1999 to 19:1, more preferably 1:199 to 19:1, still more preferably 1:19 to 3:1, further preferably 1:2 to 2:1. If the weight ratio of the active pharmaceutical ingredient and the excipient is outside the above ranges, uniform mixing is difficult, resulting in a reduced pharmacological effect or a tendency to be difficult to freeze-dry.

The fine particles (b) have a mean particle size of preferably 0.8 μm or more and 5.0 μm or less, more preferably 1.0 μm or more and 4.0 μm or less, still more preferably 2.0 μm or more and 3.5 μm or less. The fine particles (b) having a mean particle size within the above ranges can be easily separated from the carrier after administering the inhalation powder, facilitating the delivery to the bronchi or alveoli.

The fine particles (b) are present on the surfaces of the carrier particles (a) preferably with the adhesion ability

based on the intermolecular interaction with the carrier particles (a). Such an inhalation powder can be obtained by mixing the carrier particles (a) with the fine particles (b). They may be mixed using a common mixer. Examples of the mixer include a horizontal cylindrical mixer, a V-type mixer, a horizontal conical mixer, a double conical mixer, a cubic mixer, a screw mixer, an orbiting screw mixer, a ribbon mixer, a rotary disc mixer, a media agitation mill, a high-speed rotary friction mill, a high-speed rotary impact mill, an impact mill, and a jet mill.

The weight ratio of the carrier particles (a) and the fine particles (b) in the inhalation powder is preferably 1:1 to 99:1, more preferably 4:1 to 19:1, still more preferably 5:1 to 9:1. If the proportion of the carrier particles (a) is outside the above ranges, the pharmacological activity may be lost. If the proportion of the fine particles (b) is outside the above ranges, the fluidity of the resulting preparation tends to decrease.

The percentage of the fine particles (b) in the inhalation powder is preferably 1% by weight or more and 50% by weight or less, more preferably 5% by weight or more and 20% by weight or less, still more preferably 9% by weight or more and 12.5% by weight or less. When the percentage is within the above ranges, the fine particles (b) tend to uniformly adhere to the surfaces of the carrier particles (a) while maintaining the pharmacological activity.

The ratio (mean particle size of fine particles (b):mean particle size of carrier particles (a)) between the mean particle sizes of the fine particles (b) and the carrier particles (a) is preferably 1:6 to 1:50, more preferably 1:10 to 1:20.

<Target disease>

The inhalation powder of the present invention can be used as a therapeutic agent for diseases corresponding to the active pharmaceutical ingredient. Examples of the target diseases include asthma (bronchial asthma), asthmatic bronchitis, chronic bronchitis, chronic obstructive pulmonary disease (COPD), allergic rhinitis, refractory chronic cough, diabetes, hypoglycemia, autoimmune diseases, and cancers.

<Deposition rate in bronchi and lungs>

The deposition rate of the inhalation powder in the bronchi and the lungs can be evaluated using an Andersen cascade impactor (ACI). The inhalation performance evaluation using an Andersen cascade impactor can be performed in accordance with the United States Pharmacopeia (USP29<601> AEROSOLS, NASAL SPRAYS, METERED-DOSE INHALERS, AND DRY POWDER INHALERS). Specific examples of the measurement device include an equipment "AN-200" available from Sibata Scientific Technology Ltd.

In the Andersen cascade impactor, the inhalation powder is separated into eight stages, specifically stage #0 to stage #7, from top to bottom, and the bottom filter, while progressively smaller particles are classified and captured aerodynamically towards the lower stages. The amount of the inhalation powder captured in each stage can be measured by a known method such as weight measurement and measurement of the active pharmaceutical ingredient by liquid chromatography. The particle size of the inhalation powder captured in each stage is as follows.

Stage #0: 11.0 μm-
Stage #1: 7.0 μm-11.0 μm
Stage #2: 4.7 μm-7.0 μm
Stage #3: 3.3 μm-4.7 μm
Stage #4: 2.1 μm-3.3 μm
Stage #5: 1.1 μm-2.1 μm
Stage #6: 0.65 μm-1.1 μm
Stage #7: 0.43 μm-0.65 μm
Filter: -0.43 μm

The inhalation powder of the present invention preferably has a total deposition rate in stages #4 to #7 of 10% or more in an inhalation performance evaluation using an Andersen cascade impactor. The total deposition rate is more preferably 20% or more, still more preferably 38% or more, further preferably 41% or more, particularly preferably 44% or more. Deposition at stages #4 to #7 is considered to indicate the deposition of the inhalation powder in the bronchi and alveoli. The total deposition rate in stages #4 to #7 can be calculated by the following <Formula 1>.

Total deposition rate (%) in stages #4 to #7 = Total amount (g) of inhalation powder captured in stages #4 to #7/Total amount (g) of inhalation powder captured in stages #0 to #7 × 100 ··· Formula 1     Formula 1

The inhalation powder of the present invention preferably has an emitted dose (ED) of preferably 50% or more in an inhalation performance evaluation using an Andersen cascade impactor. The ED is more preferably 60% or more, still

more preferably 70% or more. The ED can be calculated by the following <Formula 2>.

ED (%) = Amount (g) of inhalation powder released from capsule/Amount (g) of inhalation powder charged in capsule × 100 ⋯ Formula 2 Formula 2

The inhalation powder of the present invention preferably has a fine particle fraction (FPF) of preferably 10% or more in an inhalation performance evaluation using an Andersen cascade impactor. The FPF is more preferably 30% or more, still more preferably 50% or more. The FPF can be calculated by the following <Formula 3>.

FPF (%) = Amount (g) of inhalation powder captured in stages #2 to #7/Amount (g) of inhalation powder captured in stages #0 to #7 × 100 ⋯ Formula 3 Formula 3

Due to the use of trehalose as an excipient, the inhalation powder of the present invention exhibits excellent storage stability and an excellent deep deposition rate even after being stored under high humidity conditions. The inhalation powder of the present invention after two-week storage at a relative humidity of 75% and a temperature of 40°C preferably has a total deposition rate in stages #4 to #7 of 10% or more in an inhalation performance evaluation using an Andersen cascade impactor. The total deposition rate is more preferably 20% or more, still more preferably 40% or more.

## EXAMPLES

The present invention will be described below with reference to, but not limited to, the following examples. Hereinafter, "parts" and "%" means "parts by weight" and "% by weight", respectively, unless otherwise specified.

### (1) Grinding of trehalose

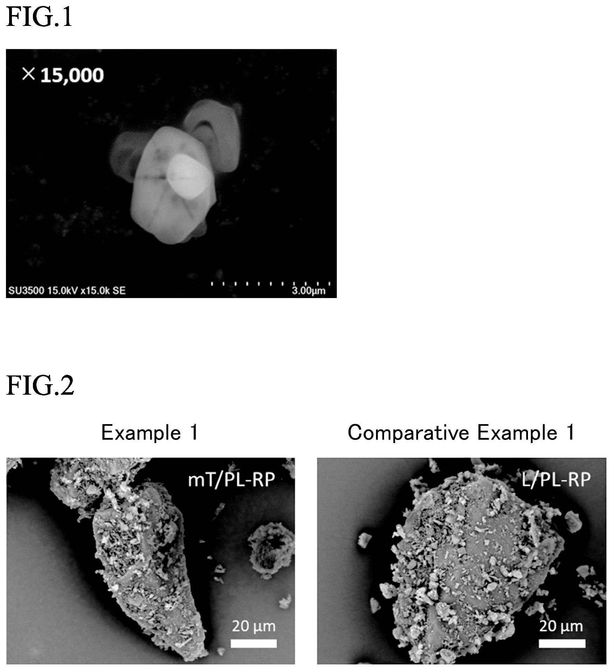

About 17 kg of pharmaceutical grade trehalose (Trehalose 100PH, manufactured and sold by Hayashibara Co., Ltd.) was ground using a jet mill (Nano Jetmizer®: NJ-100 Type C, available from Aishin Nano Technologies Co., Ltd.), thereby preparing about 13 kg of micronized trehalose having a mean particle size of 1.05 μm. The grinding conditions included a treated amount of 7.9 kg/hr, and a feeding pressure and a grinding pressure both at 1.4 MPa. The micronized trehalose was observed with a scanning electron microscope (SEM: SU3500 type, available from HITACHI, Ltd.). A small amount of a sample was placed on a sample stage, and excess particles were removed by air blowing. Observation at an accelerating voltage of 15.0 kV found particles with a diameter of about 1 μm. FIG. 1 shows the result.

### (2) Preparation of inhalation powder 1

Inhalation powders (pranlukast respirable powder (PL-RP)) including pranlukast (PL, Compound ID: AG008R1P, available from AngeneInternational Limited) as API were prepared according to the formulations indicated in Table 1. The inhalation powders were compared with each other in terms of the inhalation performance.

Composition of inhalation powder

[Table 1]

| Formulation ratio (w/w%) | Excipient | | API | Carrier |
|---|---|---|---|---|
| | Micronized trehalose | Lactose | PL | Lactose |
| mT/PL-RP (Example 1) | 5 | 0 | 5 | 90 |
| L/PL-RP (Comparative Example 1) | 0 | 5 | 5 | 90 |

An excipient, specifically pharmaceutically acceptable lactose (CAS RN®: 64044-51-5) or micronized trehalose, and PL were ground and mixed using a jet mill (A-O-Jet Mill, available from Seishin Enterprise Co., Ltd.) to obtain a uniformly mixed fine particles (Dv50 = about 3 to 4 μm). The raw materials to be ground were fed with an auto-feeder at a feeding pressure of 6.5 kg/cm$^2$G and ground at a grinding pressure of 6.5 kg/cm$^2$G. The ground product was mixed with a nine-fold amount of carrier lactose (Dv50 = 60 μm) to prepare PL-RP.

FIG. 2 shows SEM observation images of mT/PL-RP (Example 1) and L/PL-RP (Comparative Example 1). In

FIG. 2, the fine particles of the excipient and PL adhered to the carrier lactose in both Example 1 and Comparative Example 1. In the case of the L/PL-RP (Comparative Example 1), particles with a size of 5.0 μm or more and 10.0 μm or more were observed. In contrast, in the case of the mT/PL-RP using micronized trehalose (Example 1), only finer particles adhered.

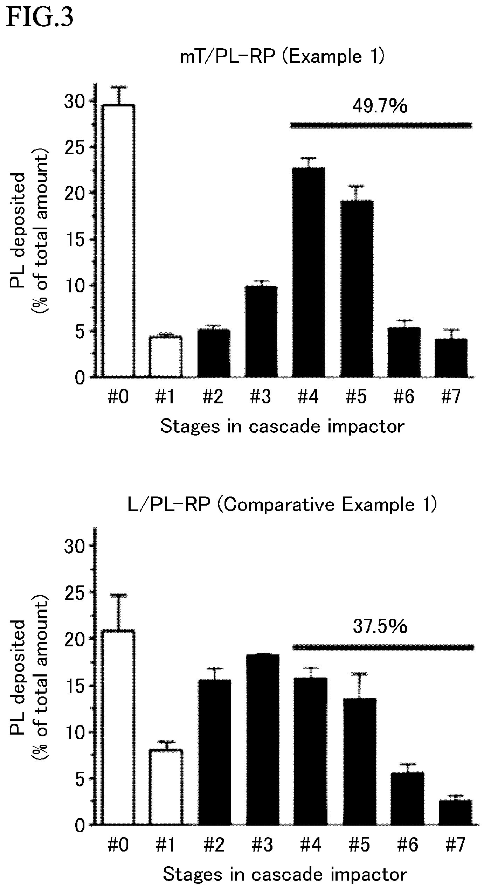

Next, the inhalation performance was evaluated using a cascade impactor. FIG. 3 shows the result, and Table 2 shows the deposition rate in each stage. About 30 mg of PL-RP was charged into a Japanese Pharmacopoeia No. 2 capsule made of hydroxypropyl methylcellulose, and the capsule was loaded into an inhaler (JetHaler®, available from Tokico System Solutions, Ltd.). After the inhaler was attached to the mouthpiece of a cascade impactor, suction was performed at a flow rate of 28.3 L/min. Subsequently, the amount of the preparation at each stage was measured by liquid chromatography.

Deposition rate in each stage

[Table 2]

| Stage No. (%) | #0 | #1 | #2 | #3 | #4 | #5 | #6 | #7 |
|---|---|---|---|---|---|---|---|---|
| mT/PL-RP (Example 1) | 30.72 | 3.92 | 4.73 | 10.93 | 25.28 | 16.99 | 4.61 | 2.82 |
| L/PL-RP (Comparative Example 1) | 20.82 | 7.96 | 15.55 | 18.22 | 15.80 | 13.59 | 5.59 | 2.47 |

The total percentage of portions delivered in stages #4 to #7 was 49.7% in the case of mT/PL-RP (Example 1) or 37.5% in the case of L/PL-RP (Comparative Example 1). The improved deep delivery rate (total percentage in stages #4 to #7) indicates that the use of trehalose is effective to deliver the API deep into the lungs.

(3) Preparation of inhalation powder 2

In order to clarify the relationship between the improved deep delivery rate confirmed in the above (2) and the particle size of the micronized trehalose, PL-RP (T/PL-RP, Example 2) was prepared using unground trehalose (untreated trehalose available from Hayashibara Co., Ltd.) as an excipient, and the inhalation performance of the PL-RP was evaluated. First, micronized trehalose or the untreated trehalose and PL were ground and mixed with a jet mill to prepare uniformly mixed fine particles (Dv50 = 3 to 4 μm). The uniformly mixed fine particles were mixed with a nine-fold amount of carrier lactose to prepare mT/PL-RP (Example 1) or T/PL-RP (Example 2). The inhalation powders in Examples 1 to 2 were prepared according to the compositions indicated in Table 3. Next, the inhalation performance was evaluated using a cascade impactor.

Composition of inhalation powder

[Table 3]

| Formulation ratio (w/w%) | Excipient | | API | Carrier |
|---|---|---|---|---|
| | Micronized trehalose | Untreated trehalose | PL | Lactose |
| mT/PL-RP (Example 1) | 5 | 0 | 5 | 90 |
| T/PL-RP (Example 2) | 0 | 5 | 5 | 90 |

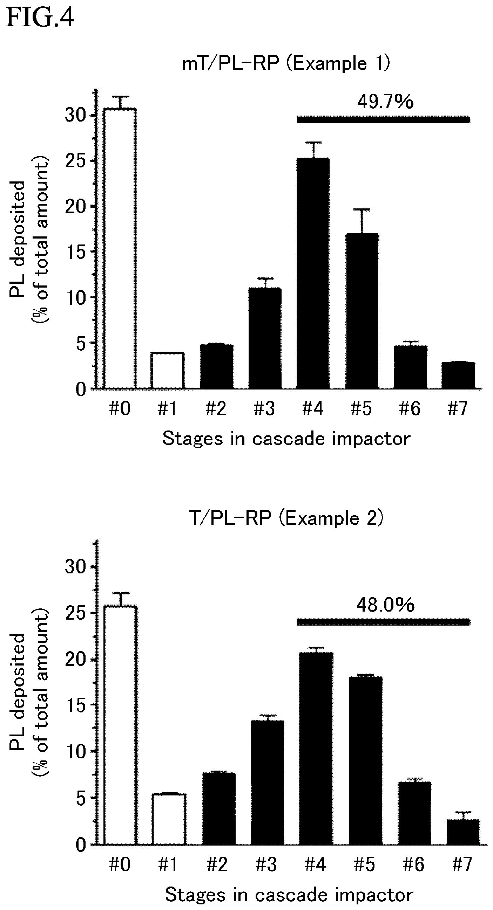

The results of the inhalation performance evaluation shown in FIG. 4 revealed that the deep delivery rate in the case of the T/PL-RP (Example 2) was improved as in the case of the mT/PL-RP (Example 1). Table 4 shows the deposition rate in each stage.

Deposition rate in each stage

[Table 4]

| Stage No. (%) | #0 | #1 | #2 | #3 | #4 | #5 | #6 | #7 |
|---|---|---|---|---|---|---|---|---|
| mT/PL-RP (Example 1) | 30.72 | 3.92 | 4.73 | 10.93 | 25.28 | 16.99 | 4.61 | 2.82 |
| T/PL-RP (Example 2) | 25.73 | 5.37 | 7.62 | 13.28 | 20.64 | 18.08 | 6.66 | 2.62 |

(4) Particle size distribution of inhalation powder

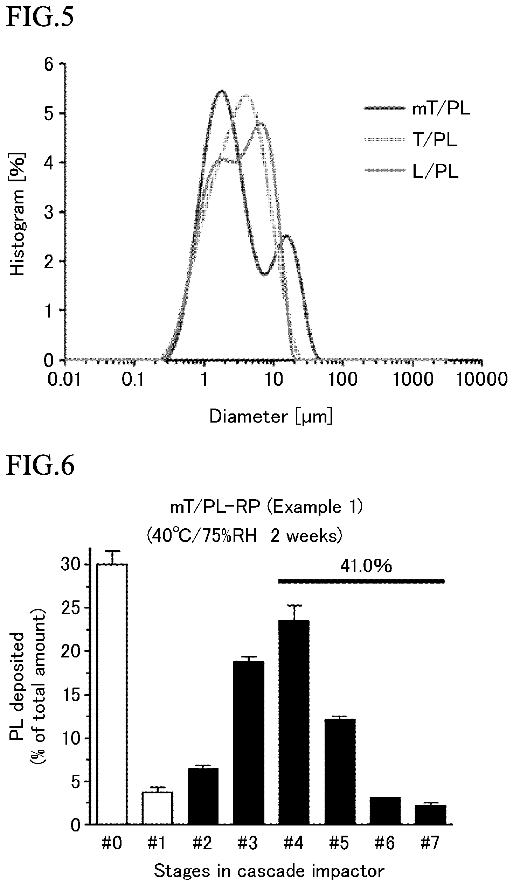

With regard to the PL-RP described in the above (2) and (3), the particle size distribution of the fine particles (before being mixed with carrier lactose) of the mixture of PL and the excipient was measured with a light scattering particle size analyzer (Mastersizer 3000 available form Malvern Panalytical). Table 5 and FIG. 5 show the result. The values in Table 5 and FIG. 5 are each a volume-based mean Dv10, Dv50, or Dv90 obtained by measuring three times under the same conditions. The particle size distribution curve was also prepared from the mean values obtained by measuring three times.

Size of fine particles of mixture of excipient and PL

[Table 5]

| | Dv10 [μm] | Dv50 [μm] | Dv90 [μm] |
|---|---|---|---|
| mT/PL | 0.91 | 2.60 | 17.40 |
| T/PL | 0.90 | 3.29 | 9.74 |
| L/PL | 0.89 | 3.37 | 10.60 |

The mean particle size (Dv50) of the T/PL was 3.29 μm, which was larger by 0.69 μm than the Dv50 of the mT/PL and almost equal to the Dv50 of the L/PL. The deep delivery rate was significantly different between Example 2 (T/PL-RP) and Comparative Example 1 (L/PL-RP) regardless of the almost equal Dv50 (see, FIG. 3 and FIG. 4). This suggests that not only the particle sizes of the API and the excipient but also the type of the sugar used affect the inhalation performance.

(5) Storage stability of inhalation powder

The mT/PL-RP (Example 1) exhibiting a high deep delivery rate was subjected to a storage stability test under high humid conditions. The mT/PL-RP was stored at a relative humidity of 75% and a temperature of 40°C for two weeks and then the inhalation performance was evaluated using a cascade impactor. FIG. 6 shows the result. As shown in FIG. 6, the total deposition rate in stages #4 to #7 was lower than the total deposition rate immediately after preparation. Still, the mT/PL-RP maintained a relatively high value as an inhalation powder.

(6) Animal test 1

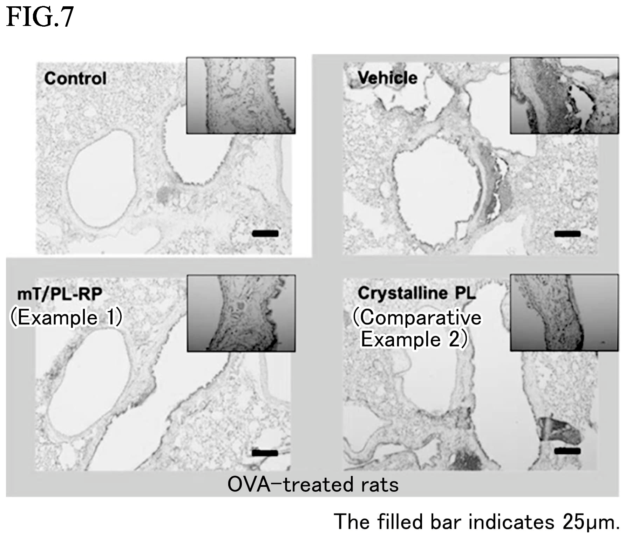

In order to evaluate the pharmacological effect of the mT/PL-RP (Example 1) via intratracheal administration, chronic obstructive pulmonary disease (COPD) was induced in rats, and the effect was compared with the effect via oral administration. Ovalbumin (OVA, Sigma-Aldrich) was intraperitoneally administered three times at seven-day intervals. Next day, the mT/PL-RP (Example 1) (5 mg-PL/kg) was intratracheally administered, or the PL (Comparative Example 2) (60 mg/kg) was orally administered. One hour after the administration, inflammation was caused by intratracheally administering micronized OVA powder to induce COPD. Twenty-four hours after the inflammation caused by OVA administration, the lungs were excised and bronchoalveolar lavage fluid (BALF) was collected. The lung tissue was stained with peroxidase by a standard method and subsequently observed. The observation revealed that the induction of COPD led to infiltration of granulocytic or monocytic cells into the lung tissue, while infiltration was suppressed in the intratracheal administration group. FIG. 7 shows the results. Although infiltration was also suppressed in the oral administration group, a higher infiltration-suppressing effect was confirmed in the intratracheal administration group.

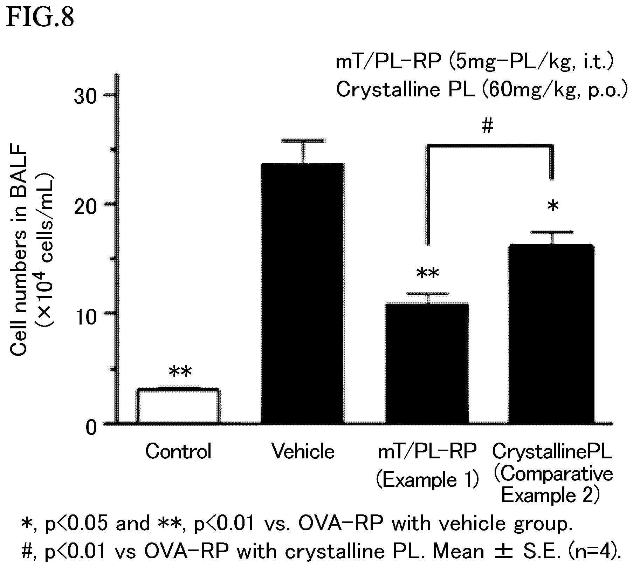

Furthermore, when the number of infiltrating cells, specifically macrophages, neutrophils, and eosinophils, in the BALF was counted using a Burker-Turk hemocytometer (Erma Inc.), the intratracheal administration group exhibited a statistically significantly lower cell count compared to the oral administration group. FIG. 8 and Table 6 show the result.

Infiltrating cell count in BALF

[Table 6]

| | Inflammatory cells in BALF ( × $10^4$ cells/mL) | | |
|---|---|---|---|
| | **Macrophages** | **Neutrophils** | **Eosinophils** |
| **Control** | **0.6 ± 0.3**** | **2.1 ± 0.7**** | **0.5 ± 0.4** |
| ***OVA-challenge*** | | | |
| **Vehicle** | **6.5 ± 1.0** | **16.2 ± 4.4** | **0.9 ± 0.6** |
| **mT/PL-RP *(i.t.)*** | **2.3 ± 0.6**** | **8.0 ± 1.7*** | **0.6 ± 0.3** |
| **Crystalline PL (p.o.)** | **3.1 ± 1.3**** | **11.5 ± 2.7** | **1.5 ± 0.6** |

*, ***p*<0.05 and **, *p*<0.01 *vs.* OVA-RP with vehicle group.**
*, ***p<0.05 vs* OVA-RP with crystalline PL. Mean±S.E. (n=4).**

In asthma, eosinophilic infiltration predominantly increases, whereas in COPD or severe asthma, neutrophilic infiltration increases. In this test, neutrophilic infiltration was predominant, indicating that the condition was similar to that of COPD. Based on these results, pulmonary administration of PL is expected to allow for dose reduction and a higher pharmacological effect compared to oral administration.

(7) Animal test 2

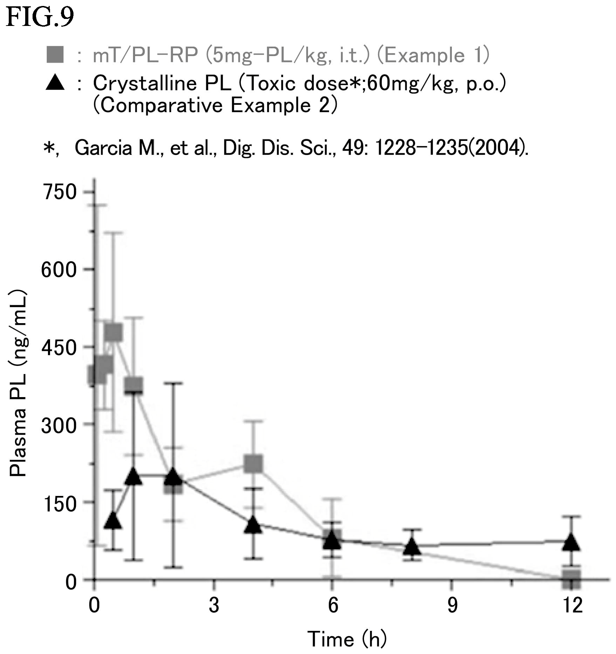

The amount of PL transferred into blood was evaluated in oral administration and intratracheal administration. The blood PL concentrations up to 12 hours after administration were measured over time in a group of rats having undergone intratracheal administration of the mT/PL-RP (Example 1) (5 mg-PL/kg) and in a group of rats having undergone oral administration of the PL (Comparative Example 2) (60 mg/kg). FIG. 9 and Table 7 show the results. The intratracheal administration group (mT/PL-RP, Example 1) exhibited faster absorption than the oral administration group (PL, Comparative Example 2) and also exhibited about 1.4 times the area under the curve (AUC) of the oral administration group at 12 hours after the administration.

Pharmacokinetics of PL

[Table 7]

| | **$C_{max}$ (ng/mL)** | **$T_{max}$ (h)** | **$AUC_{0\text{-}12\ h}$ (ng.h/mL)** |
|---|---|---|---|
| **mT/PL-RP (i.t.)** | **770±270** | **0.3 ± 0.2** | **1,700±950** |
| **Crystalline PL (*p.o.*)** | **250 ± 160** | **1.6 ± 0.6** | **1.200±710** |

**Mean±S.E. (*n*=4-5).**

(8) Preparation of inhalation powder 3

Theophylline (THE: product code T0179, sold by Tokyo Chemical Industry Co., Ltd.), which is a bronchial asthma therapeutic agent like PL, and an excipient were once dissolved and freeze-dried (FD) to prepare an inhalation powder (FD/THE-RP). The inhalation performance of the inhalation powder was evaluated. Specifically, THE and an excipient (trehalose or lactose) were dissolved in Milli-Q water at a weight ratio of 1:1, frozen at -20°C, and then freeze-dried using an FDU-1200 (available from Tokyo Rika Kikai Co., Ltd.). Next, the resulting product was processed with a jet mill into fine particles and then mixed with a nine-fold amount of carrier lactose to prepare FD-T/THE-RP (Example 3) and FD-L/THE-RP (Comparative Example 3) according to the compositions indicated in Table 8.

Composition of inhalation powder

[Table 8]

| Formulation ratio (w/w%) | Excipient | | API | Carrier |
|---|---|---|---|---|
| | Trehalose | Lactose | THE | Lactose |
| FD-T/THE-RP (Example 3) | 5 | 0 | 5 | 90 |
| FD-L/THE-RP (Comparative Example 3) | 0 | 5 | 5 | 90 |

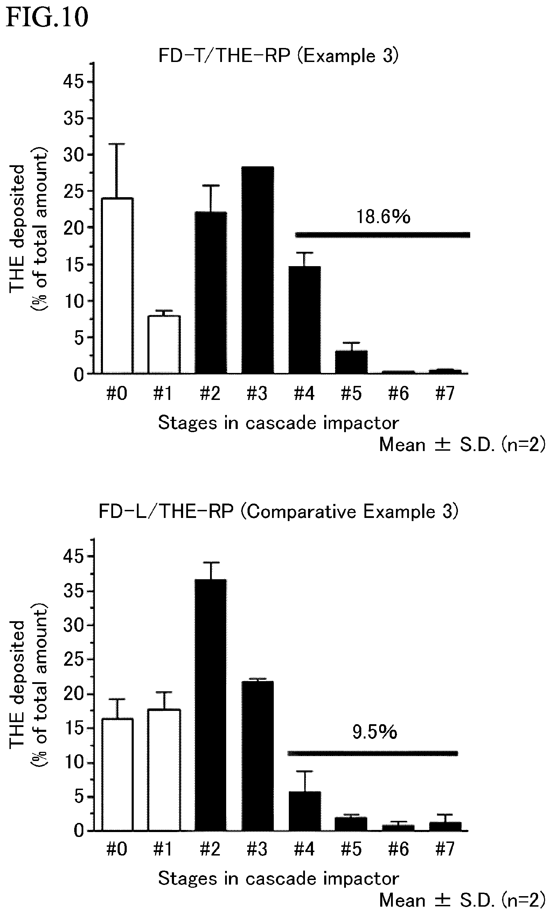

The result of comparison of the inhalation performance using a cascade impactor revealed that the deposition rate of the FD-T/THE-RP (Example 3) and the deposition rate of the FD-L/THE-RP (Comparative Example 3) in stage #4 and subsequent stages were 18.6% and 9.5%, respectively, indicating that the FD-T/THE-RP (Example 3) exhibited a two times higher deep delivery rate. FIG. 10 and Table 9 show the result.

Deposition rate in each stage

[Table 9]

| Stage No. (%) | #0 | #1 | #2 | #3 | #4 | #5 | #6 | #7 |
|---|---|---|---|---|---|---|---|---|
| FD-T/THE-RP (Example 3) | 23.95 | 7.99 | 22.15 | 28.18 | 14.66 | 3.07 | 0.31 | 0.51 |
| FD-L/THE-RP (Comparative Example 3) | 16.39 | 17.76 | 36.53 | 21.71 | 5.66 | 1.93 | 0.70 | 1.22 |

Usually, inhalation powders prepared through freeze-drying have high adhesiveness and tend to present challenges in inhalation performance. The test result suggested a potential effect of trehalose in maintaining the inhalation performance of an inhalation powder prepared through freeze-drying.

(9) Preparation of inhalation powder 4

An amount of 26 mL of an antibody solution containing 21.44 mg of a mouse anti-human IL-18 monoclonal antibody, 387 mg of sodium phosphate, and 263 mg of glycine hydrochloride was prepared. The antibody solution was freeze-dried and then mixed with an excipient (trehalose or lactose) at a weight ratio indicated in Table 10. The mixture was ground using a jet mill to prepare fine particles. The fine particles were mixed with carrier lactose (Dv50 = 60 μm) in an amount indicated in Table 10, thereby preparing T/Ab-RP (Example 4) or L/Ab-RP (Comparative Example 4) containing the antibody as an active pharmaceutical ingredient.

[Table 10]

| Formulation ratio (w/w%) | Excipient | | API | Carrier |
|---|---|---|---|---|
| | Trehalose | Lactose | Ab/salt | Lactose |
| T/Ab-RP (Example 4) | 8.9 | 0 | 0.2 | 90.9 |
| L/Ab-RP (Comparative Example 4) | 0 | 8.9 | 0.2 | 90.9 |

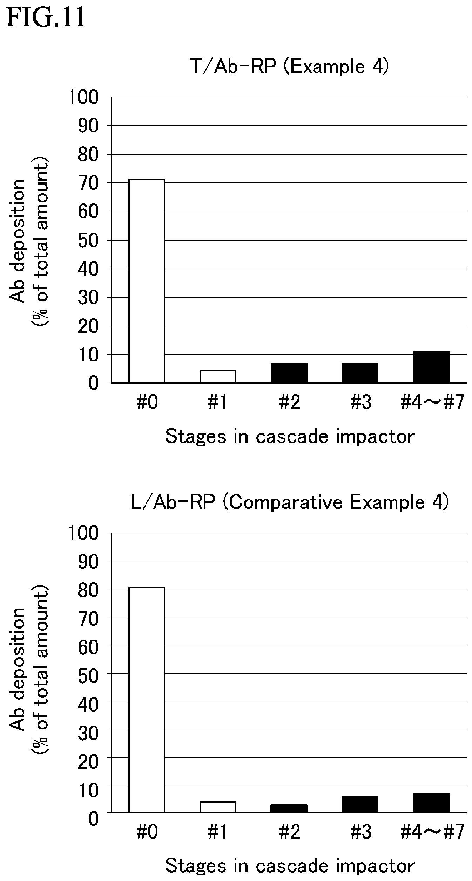

The inhalation powder was suctioned using a cascade impactor, and the weight of the inhalation powder deposited in each stage was measured. The result revealed that the deposition rate of the T/Ab-RP (Example 4) and the L/Ab-RP (Comparative Example 4) in stage #4 and subsequent stages were 11.1% and 6.8%, respectively, indicating that the T/Ab-RP (Example 4) exhibited about a 1.6 times higher deep deliberate rate than Comparative Example 4. FIG. 11 and Table 11 show the result.

[Table 11]

| Stage No. (%) | #0 | #1 | #2 | #3 | Total of #4 to #7 |
|---|---|---|---|---|---|
| T/Ab-RP (Example 4) | 71.11 | 4.44 | 6.67 | 6.67 | 11.11 |
| L/Ab-RP (Comparative Example 4) | 80.67 | 3.98 | 2.84 | 5.69 | 6.82 |

The result demonstrated that the use of trehalose increased the deep delivery rate of even an inhalation powder including an antibody as an active pharmaceutical ingredient.
The present disclosure may include the following embodiments.
(Clause 1) An inhalation powder, including:

carrier particles (a); and
fine particles (b) present on surfaces of the carrier particles, the fine particles (b) including an active pharmaceutical ingredient and an excipient,
the excipient including trehalose.

(Clause 2) The inhalation powder according to clause 1,
wherein the fine particles (b) have a mean particle size of 0.8 μm or more and 5.0 μm or less.
(Clause 3) The inhalation powder according to clause 1 or 2,
wherein the carrier particles (a) include at least one of lactose or trehalose.
(Clause 4) The inhalation powder according to any one of clauses 1 to 3,
wherein the carrier particles (a) have a mean particle size of 30 μm or more and 300 μm or less.
(Clause 5) The inhalation powder according to any one of clauses 1 to 4,
wherein the active pharmaceutical ingredient is selected from the group consisting of a low molecular weight compound, a peptide, and a protein.
(Clause 6) The inhalation powder according to clause 5,
wherein the low molecular weight compound is selected from the group consisting of a leukotriene receptor antagonist and a xanthine derivative.
(Clause 7) The inhalation powder according to clause 6,
wherein the leukotriene receptor antagonist is pranlukast, or the xanthine derivative is theophylline.
(Clause 8) The inhalation powder according to clause 5,
wherein the peptide or the protein is glucagon or an antibody.
(Clause 9) The inhalation powder according to any one of clauses 1 to 8,
wherein the inhalation powder has a total deposition rate in stages #4 to #7 of 10% or more in an inhalation performance evaluation using an Andersen cascade impactor.
(Clause 10) The inhalation powder according to any one of clauses 1 to 9,
wherein the fine particles (b) are prepared by drying a mixture of the active pharmaceutical ingredient and the excipient.
(Clause 11) The inhalation powder according to any one of clauses 1 to 10,
wherein the weight ratio of the active pharmaceutical ingredient and the excipient is 1:1999 to 19:1.
(Clause 12) The inhalation powder according to any one of clauses 1 to 11,
wherein the weight ratio of the carrier particles (a) and the fine particles (b) is 1:1 to 99:1.

## Claims

1. An inhalation powder, comprising:

   carrier particles (a); and
   fine particles (b) present on surfaces of the carrier particles, the fine particles (b) including an active pharmaceutical ingredient and an excipient,
   the excipient including trehalose.

2. The inhalation powder according to claim 1,
   wherein the fine particles (b) have a mean particle size of 0.8 μm or more and 5.0 μm or less.

3. The inhalation powder according to claim 1 or 2,
   wherein the carrier particles (a) include at least one of lactose or trehalose.

4. The inhalation powder according to claim 1 or 2,
   wherein the carrier particles (a) have a mean particle size of 30 μm or more and 300 μm or less.

5. The inhalation powder according to claim 1 or 2,
   wherein the active pharmaceutical ingredient is selected from the group consisting of a low molecular weight compound, a peptide, and a protein.

**6.** The inhalation powder according to claim 5,
wherein the low molecular weight compound is selected from the group consisting of a leukotriene receptor antagonist and a xanthine derivative.

**7.** The inhalation powder according to claim 6,
wherein the leukotriene receptor antagonist is pranlukast, or the xanthine derivative is theophylline.

**8.** The inhalation powder according to claim 5,
wherein the peptide or the protein is glucagon or an antibody.

**9.** The inhalation powder according to claim 1 or 2,
wherein the inhalation powder has a total deposition rate in stages #4 to #7 of 10% or more in an inhalation performance evaluation using an Andersen cascade impactor.

**10.** The inhalation powder according to claim 1 or 2,
wherein the fine particles (b) are prepared by drying a mixture of the active pharmaceutical ingredient and the excipient.

**11.** The inhalation powder according to claim 1 or 2,
wherein the weight ratio of the active pharmaceutical ingredient and the excipient is 1:1999 to 19:1.

**12.** The inhalation powder according to claim 1 or 2,
wherein the weight ratio of the carrier particles (a) and the fine particles (b) is 1:1 to 99:1.

FIG.1

×15,000
SU3500 15.0kV x15.0k SE
3.00μm

FIG.2

Example 1

mT/PL-RP
20 μm

Comparative Example 1

L/PL-RP
20 μm

FIG.3

mT/PL-RP (Example 1)
49.7%
PL deposited
(% of total amount)
30
25
20
15
10
5
0
#0
#1
#2
#3
#4
#5
#6
#7
Stages in cascade impactor

L/PL-RP (Comparative Example 1)
37.5%
PL deposited
(% of total amount)
30
25
20
15
10
5
0
#0
#1
#2
#3
#4
#5
#6
#7
Stages in cascade impactor

# FIG.4

mT/PL-RP (Example 1)
49.7%
PL deposited
(% of total amount)
0
5
10
15
20
25
30
#0
#1
#2
#3
#4
#5
#6
#7
Stages in cascade impactor

T/PL-RP (Example 2)
48.0%
PL deposited
(% of total amount)
0
5
10
15
20
25
30
#0
#1
#2
#3
#4
#5
#6
#7
Stages in cascade impactor

FIG.5

6
5
4
3
2
1
0
Histogram [%]
0.01
0.1
1
10
100
1000
10000
Diameter [μm]
mT/PL
T/PL
L/PL

FIG.6

mT/PL-RP (Example 1)
(40°C/75%RH 2 weeks)
41.0%
30
25
20
15
10
5
0
PL deposited
(% of total amount)
#0
#1
#2
#3
#4
#5
#6
#7
Stages in cascade impactor

FIG.7

Control
Vehicle
mT/PL-RP
(Example 1)
Crystalline PL
(Comparative
Example 2)
OVA-treated rats

The filled bar indicates 25μm.

FIG.8

mT/PL-RP (5mg-PL/kg, i.t.)
Crystalline PL (60mg/kg, p.o.)
#
Cell numbers in BALF
(×10⁴ cells/mL)
30
20
10
0
**
*
**
Control
Vehicle
mT/PL-RP
(Example 1)
CrystallinePL
(Comparative
Example 2)

*, $p<0.05$ and **, $p<0.01$ vs. OVA-RP with vehicle group.
#, $p<0.01$ vs OVA-RP with crystalline PL. Mean ± S.E. (n=4).

FIG.9

■ : mT/PL-RP (5mg-PL/kg, i.t.) (Example 1)

▲ : Crystalline PL (Toxic dose*;60mg/kg, p.o.) (Comparative Example 2)

*, Garcia M., et al., Dig. Dis. Sci., 49: 1228-1235(2004).

750
600
450
300
150
0
Plasma PL (ng/mL)
0
3
6
9
12
Time (h)

# FIG.10

FD-T/THE-RP (Example 3)
THE deposited
(% of total amount)
45
35
30
25
20
15
10
5
0
18.6%
#0
#1
#2
#3
#4
#5
#6
#7
Stages in cascade impactor
Mean ± S.D. (n=2)

FD-L/THE-RP (Comparative Example 3)
THE deposited
(% of total amount)
45
35
30
25
20
15
10
5
0
9.5%
#0
#1
#2
#3
#4
#5
#6
#7
Stages in cascade impactor
Mean ± S.D. (n=2)

# FIG.11

T/Ab-RP (Example 4)
Ab deposition
(% of total amount)
100
90
80
70
60
50
40
30
20
10
0
#0
#1
#2
#3
#4～#7
Stages in cascade impactor

L/Ab-RP (Comparative Example 4)
Ab deposition
(% of total amount)
100
90
80
70
60
50
40
30
20
10
0
#0
#1
#2
#3
#4～#7
Stages in cascade impactor

## INTERNATIONAL SEARCH REPORT

International application No. **PCT/JP2024/037613**

**A. CLASSIFICATION OF SUBJECT MATTER**

***A61K 9/72***(2006.01)i; ***A61K 9/14***(2006.01)i; ***A61K 9/16***(2006.01)i; ***A61K 31/41***(2006.01)i; ***A61K 31/522***(2006.01)i; ***A61K 38/26***(2006.01)i; ***A61K 39/395***(2006.01)i; ***A61K 45/00***(2006.01)i; ***A61K 47/26***(2006.01)i; ***A61P 3/10***(2006.01)i; ***A61P 11/00***(2006.01)i; ***A61P 11/02***(2006.01)i; ***A61P 11/06***(2006.01)i; ***A61P 35/00***(2006.01)i; ***A61P 37/02***(2006.01)i; ***A61P 37/08***(2006.01)i; ***A61P 43/00***(2006.01)i

FI: A61K9/72; A61K9/14; A61K31/41; A61K31/522; A61K38/26; A61K39/395; A61K39/395 M; A61K45/00; A61K47/26; A61P3/10; A61P11/00; A61P11/02; A61P11/06; A61P35/00; A61P37/02; A61P37/08; A61P43/00 111; A61K9/16

According to International Patent Classification (IPC) or to both national classification and IPC

**B. FIELDS SEARCHED**

Minimum documentation searched (classification system followed by classification symbols)

A61K9/72; A61K9/14; A61K9/16; A61K31/41; A61K31/522; A61K38/26; A61K39/395; A61K45/00; A61K47/26; A61P3/10; A61P11/00; A61P11/02; A61P11/06; A61P35/00; A61P37/02; A61P37/08; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2001/026630 A1 (KAKEN PHARMACEUTICAL CO., LTD.) 19 April 2001 (2001-04-19)<br>claims, p. 12, lines 16-23, p. 7, lines 10-13, p. 8, lines 16-22, examples 9-11, 19, 21, 23 | 1-12 |
| X | JP 2010-509287 A (BOEHRINGER INGELHEIM PHARMA GMBH & CO. KG) 25 March 2010 (2010-03-25)<br>claims, paragraphs [0011], [0025], [0033], examples 1-2 | 1-5, 8-12 |
| A | JP 2001-072586 A (NIKKEN CHEM CO., LTD.) 21 March 2001 (2001-03-21)<br>examples 1-2, test example 1 | 1-12 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2024** | **07 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/037613**

| Patent document cited in search report | Publication date (day/month/year) | Patent family member(s) | Publication date (day/month/year) |
|---|---|---|---|
| WO 2001/026630 A1 | 19 April 2001 | US 7022311 B1<br>claims 1-19, p. 7, lines 32-42, p. 4, lines 51-56, p. 5, lines 26-37, examples 9-11, 19, 21, 23<br>EP 1238661 A1<br>JP 4691298 B1 | |
| JP 2010-509287 A | 25 March 2010 | US 2010/0143331 A1<br>claims 1-39, paragraphs [0036], [0103]-[0104], [0128], examples 1-2<br>WO 2008/055951 A1<br>EP 2091518 A1<br>KR 10-2009-0082476 A | |
| JP 2001-072586 A | 21 March 2001 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001072586 A